# EUROPEAN PATENT SPECIFICATION

(11) **EP 2 332 647 B1**
(45) Date of publication and mention of the grant of the patent: **13.06.2018**
(21) Application number: 10194156.5
(22) Date of filing: 08.12.2010
(51) Int. Cl.: B01J 23/882, C07C 2/24, C10G 50/00, B01J 37/34, B01J 35/00

(54) **Nanocatalyst for conversion of monoolefins, process for conversion of monoolefins and process for preparing catalyst**
Nanokatalysator zur Umwandlung von Monoolefinen, Verfahren zur Umwandlung von Monoolefinen und Verfahren zur Herstellung eines Katalysators
Nano-catalyseur pour la conversion de mono-oléfines, procédé de conversion de mono-oléfines et procédé de préparation de catalyseur

(30) Priority: 09.12.2009 EP 09015244
(43) Date of publication of application: 15.06.2011
(73) Proprietor: King Abdulaziz City for Science and Technology, Riyadh 11442 (SA)
(72) Inventor: Al-Kinany, Mohammed, 11442, Riyadh (SA); Al-Khowaiter, Soliman, 11442, Riyadh (SA); Al-Drees, Saud, 11442, Riyadh (SA); Alshehry, Feras, 11442, Riyadh (SA); Al-Rasheed, Rasheed, 11442, Riyadh (SA)
(74) Representative: Goddar, Heinz J.

(56) References cited:
- WO-A1-2004/050238
- US-A- 3 642 661
- US-A- 4 720 477
- US-A1- 2006 063 955
- US-A1- 2007 066 480
- US-A1- 2007 235 187
- US-A1- 2008 217 008
- US-B1- 6 884 916
- CHOI K-H ET AL: "Preparation and characterization of nano-sized CoMo/Al2O3 catalyst for hydrodesulfurization", APPLIED CATALYSIS A: GENERAL, ELSEVIER SCIENCE, AMSTERDAM, NL, vol. 260, no. 2, 8 April 2004 (2004-04-08) , pages 229-236, XP004500659, ISSN: 0926-860X, DOI: DOI:10.1016/J.APCATA.2003.10.019
- LANDAU M V ET AL: "Ultrasonically Controlled Deposition-Precipitation - Co-Mo HDS Catalysts Deposited on Wide-Pore MCM Material", JOURNAL OF CATALYSIS, ACADEMIC PRESS, DULUTH, MN, US, vol. 201, no. 1, 1 July 2001 (2001-07-01), pages 22-36, XP004432508, ISSN: 0021-9517, DOI: DOI:10.1006/JCAT.2001.3227

## Description

The present invention relates to a process for oligomerisation of monoolefins in the presence of a nanocatalyst.

Especially, the nanocatalyst is suitable to be used in a process for conversion of monoolefins into oligomers that can be used as clean fuel distillates in the range of gasoline, jet fuel and diesel.

It is known that gasoline, jet fuel and diesel are the most important fuels used in all kinds of transportations. The three types of fuel are often produced by fractionated distillation of crude oil under atmospheric pressure and other refinery processes. There are several types of gasoline produced in refineries depending on the quality of crude oil, refinery processes and units used. The most important fuels are as follows:
a) Natural gasoline: Natural gasoline is a mixture of volatile hydrocarbon compounds having from 4 to 6 carbon atoms - this type of gasoline may contain hydrogen sulfide and some mercaptanes. It is not the final product produced for consumption, but is mixed with other fuel or is reprocessed to improve its combustion properties.
b) Crude gasoline: Crude gasoline is produced from the process of distillation of crude oil under atmospheric pressure. The number of carbon atoms is in the range of from 4 to 9. It is used as fuel after improvement of its combustion properties. Crude gasoline is divided into two types:
   - Light gasoline: It is produced from direct distillation of crude oil. Its octane number and the sulphur content, which is up to about 1000 ppm, depend on the specification of the crude oil used.
   - Heavy gasoline: The sulphur content of this type of gasoline is up to about 1500 ppm. It is subjected to a reforming process to raise its octane number; and to hydrotreatment to reduce or remove the sulphur content.
c) Jet fuel: Jet fuel is also known as kerosene. It is composed of a mixture of hydrocarbon compounds consisting of carbon atoms ranging from 6 to 16 atoms. This type of fuel is mixed with gasoline for use as fuel in aircrafts. Based on the purpose it is used for, there are different kinds thereof. The sulphur content of these types of fuel depends on the type of crude oil. Jet fuel is also subjected to hydrodesulphurization for reducing or removing sulphur contents.
d) Diesel fuel: Diesel fuel is a mixture of straight chain and/or branched hydrocarbon compounds, naphthenics and aromatics. The number of carbon atoms is from 10 to 22 carbon atoms. Diesel fuel is produced from the distillation of crude oil under atmospheric pressure, and from cracking processes. Dieses fuel also contains sulphur and nitrogen compounds. In order to produce high quality diesel fuel, sulphur, nitrogen and aromatic compounds should be removed by hydrogen treatment processes.

Improvements of the combustion properties of the fuels mentioned above require a set of processes.

Various types of catalysts and reactors are used in this regard at petroleum refineries, under strict environmental laws. Improvements could be achieved by using traditional processes such as alkylation and isomerization to raise the octane number of gasoline and jet fuel, and cetan number for diesel. In addition, sulphur, nitrogen and (other) aromatic compounds could be removed by other processes which are well known in the art.

Conventional catalysts used so far are not effective to reduce the aromatic compounds and to remove all types of sulphur and nitrogen compounds, or to reduce the contents to less than one part per million, because the catalysts known so far have a short lifetime, deactivate quickly and cannot be recycled or re-used.

Since catalytic technology is the key for petroleum refining and petrochemical processing, and catalysts are the core of catalytic technology, the development of new catalysts would have a great impact on petroleum refining and petrochemical industry.

Nano-materials offer many possibilities as catalysts to meet future demands in the above catalytic process technology in petroleum refining, petrochemical industry and synthetic fuel production in the future. In the present application, nanomaterials are to be understood to have an average particle size in the nanometer range, for example from 25-500 nm.

Recently, nanocatalysts have attracted much attention as showing higher activity and better selectivity compared to traditional catalysts. Higher activity and better selectivity of nanocatalysts over traditional catalysts are attributed to their large specific surface area, high percentage of surface atoms and/or special crystal structures.

The development of nanocatalysts is increasingly supported by advances in preparation, characterization and testing of catalysts for the determination of property/performance relationships.

A variety of methods is known in the art to produce nanoparticles, such as wet chemistry based methods, high temperature synthesis methods or flame-based methods and spray pyrolysis.

The ban of methyl tertiary butyl ether (MTBE) from gasoline in California with its likely extension to the rest of the USA and continued attacks owing to its presumed environmental impact have in fact jeopardized its use in future reformulated gasolines.

Alkylated and dimerized products of olefins are in principle ideal compounds for reformulated gasolines, as they satisfy all the regulations owing to their combination of high octane number and low volatility as well as complete absence of sulphur and aromatic compounds.

Unfortunately, the alkylation product between isobutene and 1-butene is of low quality when sulphuric acid is used as alkylation catalyst; the quality obtained using hydrofluoric acid is slightly better, but this is generally undesirable for environmental aspects.

From environmental point of view, sulphuric acid and hydrofluoric acid are strong acids, classified among dangerous substances, owing to their corrosive liquid nature. In case of discharge into the atmosphere due to an occasional accident, hydrofluoric acid, which is extremely volatile, forms a cloud of toxic vapours, whereas sulfphuric acid remains liquid and is therefore easier to handle. However, it should be pointed out on the other hand that handling of enormous volumes of sulphuric acid in a routine operation, the disposal of its byproducts and transporting of the acid for its recovery analogously represent a great risk to environment.

As a result of these problems, new effective and selective catalysts for conversion of monoolefins, i.e. oligomerization, are highly desired.

Fuel reformulation is nowadays carried out on a world-wide basis due to US and European legislations which are focused on reducing evaporative emissions, lower sulphur content, lower aromatics and on complete fuel combustion. In this context, an interesting route for the production of environmentally friendly fuel is the dimerization, trimerization, tetramerization (known as oligomerization) of light olefins. This process is particularly attractive, since the olefin C4 fractions of the FCC process can be used as a feed, with isobutene, 1-butene and 2-butenes being the main components of this fraction. When the olefin source is isobutene, highly substituted C8 olefins can be obtained.

It has been proposed in US 2,830,106 to dimerize C6-C15 olefins, particularly alpha-olefins, in the presence of a catalyst having a base of activated alumina containing 0.5 to 3% by weight of hydrofluoric acid. Such a process has numerous drawbacks, in that only a low rate of conversion of the olefin is obtained, with a very low yield of dimers. Further, the preparation of the catalyst is very complicated.

Similarly, US 4,205,195 describes the dimerization of alpha-olefins having from 8 to 20 carbon atoms in the presence of hydrofluoric acid. In particular, this patent shows the dimerization of straight-chain olefins having a terminal double bond, preferably alpha-olefins of 10-16 carbon atoms.

A number of different catalysts have been used in the oligomerization of olefins. The oligomerization process using phosphoric acid on a silica support as catalyst has been used for several years to produce gasoline. Trimer products of isobutene oligomerization are also of interest, due to the industrial use of triisobutylene as a source of additive for gas-oil. The scientific interest and commercial importance of butene oligomerization have lead to the search for a new solid catalytic material that can avoid the formation of higher molecular weight olefins due to their controlled acidity. Amongst the catalysts reported for this reaction, other than phosphoric acid over silica, are Ziegler-Natter-based catalysts, zeolites, sulfonic resins, benzylsulfonic acid on silica, mica montmorillonite, titanium oxide and zirconium oxide.

When dimerizing olefins, it is known that heavy oligomers are generated as byproducts which gradually deactivate the catalyst.

WO2004/050238 A1 discloses a catalyst composition comprising transition metal oxides on a support. Specific, the support is colloidal silica having a particle size in the range of 8-100 nm. The catalyst has been prepared by mixing aqueous solutions of the metals with silica sol, drying and calcining. The catalyst is used in the ammoxidation of monooloefins. US 6, 884,916 B1, US 2006/063955 A1 and US 3,642,661 A discloses supported transition metal catalysts for the oligomerisation of monoolefins in the liquid phase. It is an object of the present invention to provide a process for the conversion of monoolefins which overcomes the drawbacks of the prior art, wherein the catalyst shows high activity and long lifetime and can be reactivated and reused. Further, the process shall be environmentally friendly and shall provide high conversions and selectivities. Especially, using the nanocatalyst in a process for conversion of monoolefins shall result in the provision of clean fuel distillates in the range of gasoline, jet fuel and diesel, which product shall be free of sulphur, nitrogen and aromatic compounds with high octane number and Reid pressure.

The object is achieved by a process for oligomerisation of monoolefins in a continuous gas phase using fixed bed reactor, comprising the step of oligomerizing the monoolefins in the presence of a nanocatalyst, the nanocatalyst comprising (i) at least one particulate support selected from Al2O3 and mixtures of Al2O3 and SiO2 (ii) at least one transition metal oxide, wherein the transition metal is selected from Mo and mixtures of Mo and Co, the transition metal oxide being supported on the support, wherein the average particle size determined by laser diffraction of the nanocatalyst is from 25-500 nm, the nanocatalyst being obtained by (a) preparing at least one aqueous solution of a water-soluble metal salt of a transition metal, wherein the transition metal is selected from Mo and mixtures of Mo and Co and ultrasonicating the at least one aqueous solution for 1 to 5 hours to obtain a homogeneous solution, (b) adding the at least one ultrasonicated solution to the support having an average particle size determined by laser diffraction of 15-400 nm and ultrasonicating the mixture for 1 to 12 hours to produce a paste, (c) drying the paste and then calcining it initially in an inert atmosphere and finally in an oxidative atmosphere at a temperature from 250°C and 600°C for 2 to 12 hours (d) optionally ultrasonicating the catalyst obtained in step (c) under dry conditions.

The average particle size of the inventive nanocatalyst is preferably determined by laser diffraction. The technique of laser diffraction is well known in the art and is based on the principle that particles passing through a laser beam will scatter light at an angle that is directly related to their size. As the particle size decreases, the observed scattering angle decreases logarhytmically. The observed scattering intensity is also dependent on particle sizes and diminishes, to a good approximation, in relation to the particle's cross-sectional area. Large particles therefore scatter light at narrow angles with high intensity, whereas small particles scatter at wider angles but with low intensity.

The primary measurement that has to be carried out within a laser diffraction system is the capture of the light scattering data for the particles under study. A typical system consists of a laser, a sample presentation system and a series of detectors.

The measurement of the average particle size is carried out in dry condition, i.e. after calcination and optional ultrasonating. For elemental analysis of the inventive nanocatalyst, X-ray fluorescence (XRF) was utilized which is one of the most widely used spectroscopic techniques in elemental identification and quantification. X-ray elemental analysis studies were recorded at 30 kV and room temperature using JEOL elemental analyzer JSX-3201. The transition metal is selected from Mo and mixtures of Mo and Co. The oxides of the support are selected from Al₂O₃ or mixture of Al₂O₃ and SiO₂. It is further preferred that the particle size of the support is from 15-300 nm.

Most preferred, the nanocatalyst comprises from 4 to 65% by weight, even preferred 4-30% by weight, of the transition metal oxide, based on the total weight of the support oxide.

It is preferred that the BET surface area of the nanocatalyst is about 100-150 m²/g. Measurement of the BET-surface area is well known in the art (BET = Brunauer, Emmett, Teller).

Furthermore, the pores of the catalyst have preferably an average diameter of about 55-65 Angström.

Surface area and pore size measurement studies were carried out by using micromeritics adsorption equipment of ASAP2010. All samples were degassed under vacuum at 300°C. The adsorption for nitrogen was measured at -196°C, the surface areas were calculated using the BET method based on adsorption data.

The pore-size distribution was analyzed applying the same procedure described above from desorption branch of the isotherm by the Parrett-Joyner-Halenda method.

It is preferred that the monoolefins have from 2 to 12 carbon atoms, preferably from 2 to 6 carbon atoms. The process is carried out in a continuous gas phase using fixed bed reactor, preferably under a pressure of 1- 40 atm, a temperature of 10-250°C, and /or with a weight hourly space velocity (WHSV) of about 0.1-50 h⁻¹.

It was surprisingly found that the nanocatalyst can be successfully utilized in the conversion, i.e. oligomerization, of monoolefins. The use of the catalyst results in the provision of clean fuel distillates in the range of gasoline, jet fuel and diesel which are free of sulphur, nitrogen and aromatic compounds. Further, it was found that the catalyst shows a high activity and a long lifetime, wherein the catalyst can be reactivated and reused which makes it environmental friendly. The conversion of olefins into fuel distillates reached up to 99% under economical operational conditions of temperatures, pressures and space velocities.

Preferably, the fuel distillates produced are characterized as free of sulphur, nitrogen and aromatic compounds with octane numbers ranging between 85-89 and Reid pressures ranging from 5-200 kPa.

Preferably, the particle size of the support ranges from 15 to 300 nm, preferably in the form of powder, or any other form onto which the catalytically active transition metal compound can be deposited.

The proportion of transition metal oxide nanocatalyst to the oxide support can vary, generally at least 1 weight percent of at least one transition metal oxide catalyst on the total weight of the support oxide will be employed. Generally, about 4 to about 65 weight percent of transition metal oxide catalysts will be employed, wherein about 4 to about 30 weight percent being preferred. One of the two transition metal oxide catalysts loading of about 0.1 to about 40 weight percent based on the total weight of the support is most preferred for most efficient use of high catalyst activity.

The process for conversion of monoolefins is a continuous process. For the continuous process it is convenient to work at a temperature ranging from 5 to 350°C, preferably from 10 to 250°C and a space velocity from the olefin from 0.1 to 50 h⁻¹ at any rate. The process can be carried out in the presence or absence of inert gas, such as nitrogen, helium or argon.

The level of catalytic activity for the nanocatalyst is found to be substantially higher than for the known homogeneous catalysts, such as sulphuric acid, hydrofluoric acid or phosphoric acid.

Reactants applicable for use in the process for the conversion of monoolefins are monoolefinic compounds which can (a) self-react, e.g. oligomerize, and can be (b) olefinic compounds which can react with other olefinic compounds, e.g. co-dimerize.

Self-reaction and co-dimerization shall be considered according to the present invention as "oligomerization" in general. Oligomerization shall therefore include dimerization, trimerization and tetramerization of monoolefins, either with each other or with different monoolefins.

Preferably, suitable monoolefinic compounds are those compounds having from about 2 to about 12 carbon atoms, such as ethylene, propylene, 1-butene, 2-butene, isobutylene, cis-2-butene, trans-2-butene, 1-pentene, 2-pentene, 1-hexene, 2-hexene, 3-hexene, 1-heptene, 2-heptene, 3-heptene, 4-heptene, octene isomers, nonene isomers, decene isomers, and other olefin isomers having from 11-12 carbon atoms. The olefins preferably have 2 to 12, particularly 2 to 10, more preferably 2 to 5 carbon atoms.

Resulting straight chain distillates may be capable of isomerization to form branched chain distillates.

Additional features and advantages of the subject-matter of the present invention can be taken from the following detailed description of preferred embodiments.

The nanocatalyst for conversion of monoolefins is produced based on an ultrasonic vibration technique, comprising the following steps:
i. Preparing at least one aqueous solution of a water-soluble metal salt selected from one of the transition metal elements as disclosed above for the catalytically active component of the catalyst.
ii. Ultrasoning the at least one aqueous solution for about 1 to 5 hours to obtain a homogeneous solution.
iii. Adding the at least one active solution to an inert nanoparticle support as disclosed above and ultrasonating the mixture for about 1 to 12 hours.
iv. Drying the produced paste and calcining it initially in an inert atmosphere and finally in an oxidizing atmosphere.
v. Optionally ultrasonating the produced nanocatalyst under dry conditions.

The transition metal salt for step (i) is preferably nitrate, acetate or chloride. An amount of metal salt is employed to result in a transition metal oxide weight percentage of preferably 4 to 65 weight percent based on the weight of the support oxide.

Preparation of the solution and ultrasonating thereof in step (ii) are conveniently carried out at temperatures ranging between 25 to 80°C. Ultrasonating in step (v) can be carried out at a temperature of 25 to 60°C, preferably 40°C.

The drying step (iv) is conveniently carried out at a temperature below 100°C, and preferably 80 to 90°C, under vacuum, for a time sufficient to remove water completely. The calcination of step (iv) is carried out at an atmosphere which is inert (such as helium) and then oxidizing (such as air or oxygen) at a programmable temperature from 250 to 600°C, preferably 400 to 550°C, for 2 to 12 hours.

The supported metal oxide nanocatalyst thus obtained can then be ultrasonated under dry conditions in order to avoid the possible formation of agglomerates. Zetasizer nono-particle analyzer series fitted with a 633 nm "red" laser was used for particle size measurement. Ultrasonic homogenizer was used to dispose and deagglomerate the calcined supported nanocatalyst into solvent such as methanol, ethanol, isopropanol, isobutanol, and water, or mixtures thereof. The average particle size of the final inventive nanocatalyst is in the range of 25 to 500 nm, preferably 25-400 nm.

The following examples describe the preparation of a catalyst of the present invention and a process for conversion of monoolefins to produce clean fuel distillates in the range of gasoline, jet fuel and diesel free of sulphur, nitrogen and aromatic compounds.

### Example 1

### Preparation of one supported transition metal nanocatalyst

(i) Solution (A): 4.91 g (3.97 mmol) of ammonium molybdate(VI)tetrahydrate, (Aldrich chemicals, 99.0% pure) was dissolved at room temperature in 100 ml of deionized water. The aqueous solution is stirred at room temperature for 60 minutes, then at 50°C for 2 hours until a clear solution is obtained. The resulting solution is filtered and then ultrasonated for 60 minutes.
   A respective solution (A) can be also prepared by using molybdenum(II) acetate dimer or molybdenum(II) chloride.
(ii) The heated ultrasonated mixture of (i) is added to 11.82 g (115.88 mmol) of an inert support of γ-Al₂O₃ with particle size 15 nm (BDH Laboratory Supplies). The resultant mixture is stirred for 60 minutes and ultrasonated for 12 hours at temperatures of 30-50°C until a homogeneous mixture is obtained.
   As support material also mixtures of γ-Al₂O₃ and SiO₂ in a millimolar ratio of 50:50, can be used.
(iii) The resulting mixture from (ii) is dried using a rotary evaporator under vacuum at a temperature in the range of 80 to 90°C, and then in a programmable oven under vacuum at temperatures ranging from 25 to 95°C. The mixture is then calcined at programmable temperature of 250 to 600°C, preferably from 400 to 550°C, first in helium for 1 hour, and then in air or oxygen stream for 12 hours.
(iv) The produced supported nanocatalyst is then ultrasonated under dry conditions for 3h. Zetasizer nano-particle analyzer series fitted with a 633 nm "red" laser was used for particle size measurement. Ultrasonic homogenizer was used to dispose and deagglomerate the calcined supported nanocatalyst into solvent such as methanol, ethanol, isopropanol, isobutanol, and water, or mixtures thereof. The average particle size of the prepared nanocatalyst was in the range of 25-500 nm.

### Example 2

### Preparation of two supported transition metal nanocatalyst

(i) Solution (A): 4.91 g (3.97 mmol) of ammonium molybdate(VI)tetrahydrate, (Aldrich chemicals, 99.0% pure) was dissolved at room temperature in 100 ml of deionized water. The aqueous solution is stirred at room temperature for 60 minutes, then at 50°C for 2 hours until a clear solution is obtained. The resulting solution is filtered and then ultrasonated for 60 minutes. For preparing solution (A) also molybdenum(II)acetate dimer or molybdenum(II)chloride can be used.
(ii) Solution (B): 4.94 g (16.97 mmol) of cobalt nitrate hexahydrate, (Aldrich chemicals, 99.2% pure) was dissolved at room temperature in 100 ml of deionized water. The aqueous solution is stirred at room temperature for 60 minutes, and then at 50°C for 2 hours until a clear solution is obtained. The resulting solution is filtered and then ultrasonated for 60 minutes.
   For preparing solution (B) also cobalt(II)acetate tetrahydrate or cobalt(II)chloride, can be used.
(iii) The solutions (A) and (B) are combined, and the mixture obtained is stirred at 50°C for 2 hours until a homogeneous clear solution is obtained which is ultrasonated for 3 hours at 80°C.
(iv) The heated ultrasonated mixture of (iii) is added to 11.82 g (115.88 mmol) of an inert support of γ-Al₂O₃ with particle size 15 nm (BDH Laboratory Supplies), or mixtures of γ-Al₂O₃ and SiO₂ with millimolar ratio of 50:50 can be used. The resultant mixture is stirred for 60 minutes and ultrasonated for 12 hours at temperatures of 30-50°C until a homogeneous mixture is obtained.
(v) The resulting mixture from (iv) is dried using a rotary evaporator under vacuum at a temperature in the range of 80 to 90°C, and then in a programmable oven under vacuum at temperatures ranging from 25 to 95°C. The mixture is then calcined at programmable temperature of 250 to 600°C, preferably from 400 to 550°C, first in helium for 1 hour, and then in air or oxygen stream for 12 hours.
(vi) The produced supported nanocatalyst is then ultrasonated under dry conditions for 3h. Zetasizer nano-particle analyzer series fitted with a 633 nm "red" laser was used for particle size measurement. Ultrasonic homogenizer was used to dispose and deagglomerate the calcined supported catalyst into solvent such as methanol, ethanol, isopropanol, isobutanol, and water, or mixtures thereof. The average particle size of the nanocatalyst prepared was in the range of 25-500 nm.

Nanocatalysts for conversion of monoolefins can be prepared in an identical manner utilizing suitable starting materials of the transition metal compound, such as acetates, chlorides and nitrates.

### Example 3

### Oligomerization of monoolefins

Typically the process can be carried out in a gas phase contineous process, wherein monoolefins are processed in a down flow fixed bed tube reactor over supported metal oxides nanocatalyst.

The gas phase process was performed at temperatures ranging from 5 to 350°C, wherein temperatures of 10 to 250°C are preferred. In a liquid phase batch process the temperatures may arrange from -15 to 500°C wherein temperatures of -15 to 250°C are preferred.

The reaction pressure can vary depending on the feed employed. The gas phase process was performed at a pressure ranging from 1 to about 40 atm. preferably; pressure ranging from 1 to 10 atm is employed. In liquid phase batch processes the pressure ranging from 1 to about 60 atm. The pressure of 1 to about 20 atm is preferred.

The contact time required for the reaction depends on the feed and the reaction conditions. A space velocity (WHSV) of about 0.1 to about 50 h⁻¹ could be employed. A WHSV of about 0.1 to 8 h⁻¹ is preferred.

The following table shows the reaction conditions of oligomerization and fuel distillate ranges with a conversion of feed stocks.

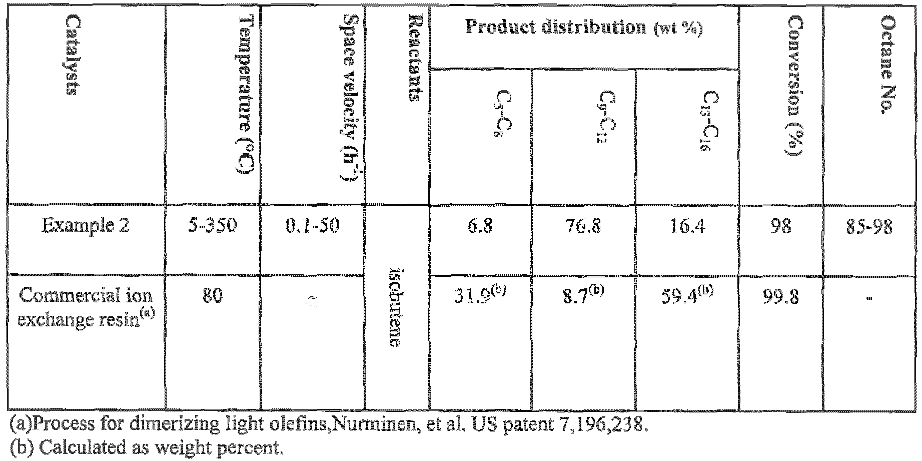

## Claims

1. Process for oligomerization of monoolefins in a continuous gas phase using fixed bed reactor, comprising the step of oligomerizing the monoolefins in the presence of a nanocatalyst, the nanocatalyst comprising
(i) at least one particulate support selected from Al2O3 and mixtures of Al2O3 and SiO2.
(ii) at least one transition metal oxide, wherein the transition metal is selected from Mo and mixtures of Mo and Co the transition metal oxide being supported on the support,
wherein the average particle size determined by laser diffraction of the nanocatalyst is from 25-500 nm, the nanocatalyst being obtained by
(a) preparing at least one aqueous solution of a water-soluble metal salt of a transition metal, wherein the transition metal is selected from Mo and mixtures of Mo and Co and ultrasonicating the at least one aqueous solution for 1 to 5 hours to obtain a homogeneous solution,
(b) adding the at least one ultrasonicated solution to the support having an average particle size determined by laser diffraction of 15-400 nm and ultrasonicating the mixture for 1 to 12 hours to produce a paste,
(c) drying the paste and then calcining it initially in an inert atmosphere and finally in an oxidative atmosphere at a temperature from 250 to 600°C for 2 to 12 hours
(d) optionally ultrasonicating the catalyst obtained in step (c) under dry conditions.

2. Process according to claim 1, wherein the monoolefins have from 2 to 12 carbon atoms.

3. Process according to claim 1 or 2, wherein the continuous gas phase process is carried out under a pressure of 1-40 atm, a temperature of 10-250°C, and/or a weight hourly space velocity (WHSV) of 0.1 to 50 h⁻¹.

4. Process according to any of the preceding claims, wherein the catalyst comprises from 4 to 65% by weight of the transition metal oxide based on the total weight of the support oxide.

5. Process according to any of the preceding claims, wherein the BET surface area of the nanocatalyst is 100-150 m²/g.

6. Process according to any of the preceding claims, wherein pores of the nanocatalyst have an average diameter of 55-65 Angström.

## Patentansprüche

1. Verfahren zur Oligomerisation von Monoolefinen in einer kontinuierlichen Gasphase unter Verwendung eines Festbettreaktors, umfassend den Schritt eines Oligomerisierens der Monoolefine in der Gegenwart eines Nanokatalysators, wobei der Nanokatalysator umfasst:
(i) wenigstens einen teilchenförmigen Träger ausgewählt aus Al₂O₃ und Mischungen von Al₂O₃ und SiO₂,
(ii) wenigstens ein Übergangsmetalloxid, wobei das Übergangsmetall ausgewählt ist aus Mo und Mischungen von Mo und Co, wobei das Übergangsmetalloxid auf dem Träger geträgert ist,
wobei die durchschnittliche Teilchengröße, bestimmt mittels Laserdiffraktion des Nanokatalysators, von 25-500 nm ist, wobei der Nanokataylsator erhalten wird durch:
(a) Herstellen wenigstens einer wässrigen Lösung eines wasserlöslichen Metallsalzes eines Übergangsmetalls, wobei das Übergangsmetall ausgewählt ist aus Mo und Mischungen von Mo und Co, und Ultraschallbehandeln der wenigstens einen wässrigen Lösung für 1 bis 5 Stunden, um eine homogene Lösung zu erhalten,
(b) Zufügen der wenigstens einen ultraschallbehandelten Lösung zu dem Träger mit einer durchschnittlichen Teilchengröße, bestimmt mittels Laserdiffraktion, von 15-400 nm und Ultraschallbehandeln der Mischung für 1 bis 12 Stunden, um eine Paste herzustellen,
(c) Trocknen der Paste und dann Kalzinieren derselben anfänglich in einer inerten Atmosphäre und letztlich in einer oxidativen Atmosphäre bei einer Temperatur von 250 bis 6oo°C für 2 bis 12 Stunden,
(d) optional Ultraschallbehandeln des in Schritt (c) erhaltenen Katalysators unter trockenen Bedingungen.

2. Verfahren nach Anspruch 1, wobei die Monoolefine von 2 bis 12 Kohlenstoffatome aufweisen.

3. Verfahren nach Anspruch 1 oder 2, wobei das kontinuierliche Gasphasenverfahren durchgeführt wird unter einem Druck von 1-40 atm, einer Temperatur von 10 bis 250°C und/oder einer Gewichtsstundenraumgeschwindigkeit (WHSV) von 0,1 bis 50 h⁻¹.

4. Verfahren nach einem der vorangehenden Ansprüche, wobei der Katalysator 4 bis 65 Gew.% des Übergangsmetalloxids, basierend auf dem Gesamtgewicht des Trägeroxids, umfasst.

5. Verfahren nach einem der vorangehenden Ansprüche, wobei die BET-Oberfläche des Nanokatalysators 100-150 m²/g ist.

6. Verfahren nach einem der vorangehenden Ansprüche, wobei Poren des Nanokatalysators einen durchschnittlichen Durchmesser von 55-65 Angström aufweisen.

## Revendications

1. Procédé d'oligomérisation de mono-oléfines dans une phase gazeuse continue en utilisant un réacteur à lit fixe, qui comprend l'étape d'oligomérisation des mono-oléfines en présence d'un nano-catalyseur, le nano-catalyseur comprenant
(i) au moins un support particulaire choisi parmi l'Al2O3 et des mélanges d'Al2O3 et de SiO2
(ii) au moins un oxyde de métal de transition, dans lequel le métal de transition est choisi parmi le Mo et des mélanges de Mo et de Co, l'oxyde de métal de transition étant supporté par le support,
dans lequel la taille moyenne des particules déterminée par diffraction par laser du nano-catalyseur est de 25-500 nm, le nano-catalyseur étant obtenu en
(a) préparant au moins une solution aqueuse d'un sel métallique hydrosoluble d'un métal de transition, dans lequel le métal de transition est choisi parmi le Mo et des mélanges de Mo et de Co, et en traitant par ultrasons ladite au moins une solution aqueuse pendant environ 1 à 5 heures afin d'obtenir une solution homogène,
(b) ajoutant ladite au moins une solution traitée par ultrasons au support présentant une taille moyenne de particules déterminée par diffraction par laser de 15-400 nm, et en traitant par ultrasons le mélange pendant 1 à 12 heures afin de produire une pâte,
(c) séchant la pâte et en la calcinant dans un premier temps sous atmosphère inerte puis sous atmosphère oxydante à une température de 250 à 600 °C pendant 2 à 12 heures,
(d) traitant éventuellement par ultrasons le catalyseur obtenu à l'étape (c) dans des conditions sèches.

2. Procédé selon la revendication 1, dans lequel les mono-oléfines comportent entre 2 et 12 atomes de carbone.

3. Procédé selon la revendication 1 ou 2, dans lequel le procédé en phase gazeuse continue est réalisé à une pression de 1-40 atm, une température de 10-250 °C, et/ou une vitesse spatiale horaire en poids (VSHP) d'environ 0,1 à environ 50 h⁻¹.

4. Procédé selon l'une quelconque des revendications précédentes, dans lequel le catalyseur comprend entre 4 et 65 % en poids de l'oxyde de métal de transition sur la base du poids total de l'oxyde de support.

5. Procédé selon l'une quelconque des revendications précédentes, dans lequel la surface BET du nano-catalyseur est de 100-150 m²/g.

6. Procédé selon l'une quelconque des revendications précédentes, dans lequel les pores du nano-catalyseur ont un diamètre moyen d'environ 55-65 angstrôms.
